# EUROPEAN PATENT APPLICATION

(11) **EP 4 541 461 A1**
(43) Date of publication of application: **23.04.2025**
(21) Application number: 23306842.8
(22) Date of filing: 20.10.2023
(51) Int. Cl.: B01L 3/00, C12M 1/00, G01N 1/28, B01L 9/00

(54) **PLATE OF CHIPS FOR MICRO-ORGANISMS LYSING**

(71) Applicant: Direct Analysis, 38000 Grenoble (FR)
(72) Inventor: LECOMTE, Emma, 38000 Grenoble (FR); TOUTAIN, Rémi, 38000 Grenoble (FR); VIRY, Thomas, 69120 VAULX-EN-VELIN (FR)
(74) Representative: Icosa

(57) **Abstract**

The present invention relates to a plate (1) comprising at least two chips (10) designed for receiving a sample to be lysed, each chip (10) featuring a fluidic network comprising fluidic interfaces (11) which includes:
- ports, including:
- an elution port (23) linked to a lysing chamber,
- a sample outlet port (22) configured for collecting a lysed sample

- ducts, including an elution duct between the elution port (23) and the sample outlet port (22), for transferring a lysed sample from the lysing chamber;

the chips (10) being aligned with an axis (10a) and regularly spaced apart so that the sample outlet port (22) of each chip (10) coincides with the axis (10a), and are spaced apart with a pitch,
characterised in that the elution duct is identical for each chip (10) of the plate (1).

## Description

### FIELD OF INVENTION

The present invention relates to the field of devices for laboratory use, in particular a plate of microfluidic chips for lysing micro-organisms.

### BACKGROUND OF INVENTION

It is known from document WO 2023/131714, in the name of the Applicant, a chip for micro-organism mechanical lysis, comprising a lysing chamber, and used in order to prepare a sample and collect the Deoxyribonucleic acid (DNA) of micro-organisms contained in said sample.

A process of lysing of a sample comprises the following steps:
- introducing a sample into the lysing chamber, through a inlet sample port, the sample (e.g., food diluted in a solution) containing micro-organisms;
- rinse the lysing chamber in order to remove unwanted elements of the sample and keep the micro-organisms only, thanks to a dedicated filter;
- apply pressure on the lysing chamber, e.g., with a pestle, in order to lyse the micro-organisms so they release their DNA;
- elude the released DNA towards a sample outlet port.

Once the DNA of the micro-organisms is ready to be collected in the sample outlet port, it is available for being collected with a pipette and sent to a polymerase chain reaction (PCR) process in order to detect, e.g., whether the micro-organisms contain infectious agents.

In the application EP 22 306 591.3 of October 20, 2022, also in the name of the Applicant, an improved chip for lysing comprises several ports in order to avoid cross contamination of the ports during the different steps of the process. On each duct linking a port to the lysing chamber, a valve is activable in order to open or to close said duct, according to the steps of the aforementioned process. E.g., during the elution step, only the duct linking the elution port to the lysing chamber and the duct linking the lysing chamber to the sample outlet port are open.

In an embodiment of WO 2023/131714 (illustrated figure 8a), a plate comprises several chips arranged in an array, in order to improve the efficiency of a laboratory when there is a need to conduct several tests. This embodiment allows a laboratory technician to collect all the samples simultaneously, with a standard multi-pipette.

Nevertheless, the individual chips of the plate provide unequal results: the Applicant has detected that on a same plate comprising several chips and testing the same sample, some results can be false-negative tests.

### SUMMARY

The aim of the invention is to overcome the drawbacks of the prior art, in particular by proposing an improved plate comprising lysing chips, wherein the chips provide equivalent, reproductible results.

Another aim of the invention is to provide a plate which is compact.

To this end, it has been developed a plate comprising at least two chips designed for receiving a sample to be lysed, each chip featuring a fluidic network comprising fluidic interfaces which includes:
- ports, including :
   - an elution port linked to a lysing chamber,
   - a sample outlet port configured for collecting a lysed sample,
- ducts, including an elution duct between the lysing chamber and the sample outlet port, for transferring a lysed sample from the lysing chamber;
the chips being disposed so that:
- the sample outlet port of each chip coincides with an axis, and
- the sample outlet ports of two adjacent chips are spaced apart with a pitch.

According to the invention, the elution duct is identical for each chip of the plate.

This allows the most critical phase of the lysing cycle, i.e., the elution of the sample, to behave in the same manner for all the chips.

Indeed, after the lysing of the sample, the DNA is liberated from the cells of the micro-organisms. In order to collect the DNA for tests purposes, it needs to be carried from the lysing chamber towards the sample outlet port, where it can be collected by a pipette.

During its carrying from the lysing chamber towards the outlet port, the DNA tends to clog or to stick against the inner walls of the elution duct. Having elution ducts of different lengths, as in the prior art, leads to different quantity of DNA lost during the elution: - the chips having shorter elution ducts provide a greater quantity of DNA, - the chips having longer elution ducts provide a smaller quantity of DNA, and these different amounts of collected DNA affect the behavior of the following PCR tests.

By consequence, detecting the sought micro-organisms with the chips having longer elution ducts is more difficult, and the following tests can be false-negative tests.

Given the elution ducts of the invention are all the same, each chip provides the same efficiency, and the same quantity of DNA in the sample outlet port.

It is to be understood that "identical ducts" means the same length of ducts, and the same section of ducts. The ducts need to behave similarly regarding the loss of DNA during the elution. Minor differences between ducts, if they do not lead to significative differences between the chips, are allowed.

In order to improve a similar behavior of each chip during the elution phase, the supplementary ducts of each chip are further identical. Indeed, the elution step comprises sending a very small quantity of fluid into the chip in order to convey the DNA, e.g., a few microliters. Considering these very small quantities, a difference of length between the ducts results in differences of pressure of the fluid, flow of the fluid, and so on. The control of the elution is tedious. Insufficient DNA may be brought to the sample outlet port if the fluid does not behave correctly.

Preferably, the fluidic networks of each chip are identical, in order to guarantee a similar behavior of each chip during each phase of the lysing cycle. The piloting of the fluidic couplers, cooperating with the chip, is eased. The whole process, not only the elution, is easier to control.

The pitch spacing two adjacent chips is preferably the pitch of a standard multi-pipette, e.g., 9mm, so as the plate is compatible with the already existing equipment of a laboratory. The same pitch separates each chip of the plate.

For the plate to be more compact, the chips are aligned with the axis and are regularly spaced apart, and a top-view projection of the chips features:
- protrusions resulting from ports, and
- concavities configured to match the protrusions of the top-view projections of an adjacent chip, in the manner of a tiling. The ports, which are functional parts of one chip, can extend over a non-functional part of one adjacent chip, optimizing the overall footprint of the plate.

In this case, a first lateral side of the chip comprises a first lateral protrusion resulting from the elution port, and a second lateral side, opposite to the first side, comprises a first lateral concavity configured to match the first lateral protrusion.

If the fluidic network of the chip comprises a discharge port, the first lateral side can further comprise a second lateral protrusion resulting from the discharge port, as well as a second lateral concavity configured to match the second lateral protrusion.

In order to further improve the compacity of the plate, the chips are arranged with a staggered layout on each side of the axis.

When the chips are arranged in a staggered layout, another overlapping of a functional part of the chip over a non-functional part of an adjacent chip can be located on a transversal side of the chip. In that case, the transversal side of the chip comprises a transversal protrusion resulting from the sample outlet port, and a transversal concavity matching the transversal protrusion. This optimization allows to divide by two the pitch between the sample outlet ports.

Advantageously, two adjacent chips are linked together by a frangible line, allowing to manually separate the two adjacent chips. This way, chips can be separated from the plate. If all the chips of a plate are not needed, a user can then separate manually only the chips he needs, without damaging them.

The chip features locating elements, configured to match a nesting platform of a machine during an automated lysing. The locating elements provide a good positioning of the plate when it is placed in the nesting platform. The locating elements being present on each chip, they also provide a good positioning of each chip that is separated from the plate.

In order to ease the positioning of the plate and the positioning of the chips, when laid on the nesting platform, the locating elements are located on a lower face of the chips.

Preferably, the locating elements are disposed on the chip with a dissymmetry pattern. This provides mistake-proofing locating elements, and avoids any wrong placement of a separated chip on the nesting platform.

To reduce the blueprint required for fluidic actuators of a machine, designed for interacting with the fluidic network of the chip, the fluidic interfaces are dispatched on both sides of a chip plane defined by the chip. Thus, the fluidic actuators can be disposed both above and under a chip plane being define by the chip which is generally planar.

In order to modify the fluidic network during the phases of the lysing, the chip comprises a valve configured to selectively open and close a duct of the fluidic network, and a valve access hole allowing the actuation of the valve. Preferably, the ports are located on an upper face of the plate, whereas the valve access hole is located on a lower face of the plate, opposite to the upper face. The ports being inlet and outlets of fluids used during the lysing process, having them all on the same side of the chip eases the manipulation of the chip, as well as the conception of the tubing of a machine designed to process the plate.

Preferably, a fluidic interface features a conical shape protruding from the chip, presenting a distal section, away from the chip, which is inferior to a proximal section, close to the chip, and the conical shape is configured to guide a fluidic actuator actuating the fluidic interface. This guiding helps the processing of the plate, in particular if processed by an automated machine.

Furthermore, when the fluidic interface is a port, the fluidic actuator is a pipe connector designed to be plugged on the port, and the conical shape is configured to seal the connection between the port and the pipe connector. This prevents any spillage during the processing of the plate, in particular if processed by an automated machine.

### BRIEF DESCRIPTION OF THE DRAWINGS

[Fig.1] is a schematic illustrating different phases of the lysing of micro-organisms, with the use of a single chip isolated from a plate according to the invention. Figure 1 reminds the main components and main phases of a mechanical lysing of a sample using a chip.
[Fig.2] is a top view of a first embodiment of a plate comprising chips aligned with an axis and regularly spaced apart with a first pitch.
[Fig.3] is a top view of a preferred embodiment of the plate, comprising chips aligned with an axis, disposed in a staggered manner, and regularly spaced apart with a second pitch.
[Fig.4] is a view in perspective, from above, of the embodiment of figure 3, illustrating frangible lines between the chips.
[Fig.5] is a view in perspective, from below, of the embodiment of figure 3, illustrating locating elements of the chips and access holes for actuating valves of the chips.
[Fig.6] is a top view of a chip separated from the plate of the preferred embodiment.
[Fig.7] is a cross section of a side view of the chip of figure 6, illustrating conic shapes of fluidic interfaces and access holes of the chip.

### DETAILED DESCRIPTION

The invention relates to a plate (1) comprising chips (10) designed for receiving a sample to be lysed, mainly characterized in that a sample outlet port (22) of each chip (10) coincides with an axis (10a), the sample outlet ports (22) of two adjacent chips (10) are spaced apart with a pitch configured to match the pitch of a multi-pipette, e.g., 9mm, and fluidic networks of each of the chips (10) are similar.

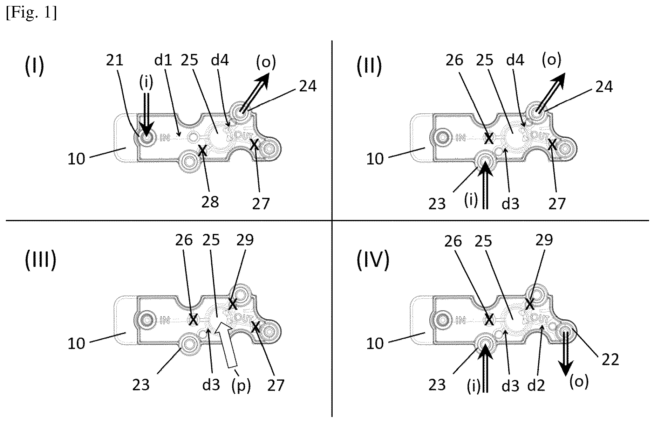

For a clarity matter, figure 1 illustrates a single chip (10) during different steps of a mechanical lysing of a sample, so that a terminology of the following description will be better understood.

A chip (10) comprises at least a sample inlet port (21), a sample outlet port (22), and a lysing chamber (25). A loading duct (d1) links the sample inlet port (21) to the lysing chamber (25), and a elution duct (d2) links the lysing chamber (25) to the sample outlet port (22).

Preferably, the chip (10) further comprises an elution port (23) and a discharge port (24). A supplementary duct (d3) links the elution port (23) to the lysing chamber (25) and a discharge duct (d4) links the lysing chamber (25) to the discharge port (24).

When the chip (10) comprises four ports (21, 22, 23, 24), each duct (d1, d2, d3, d4) comprises a valve (26, 27, 28, 29) configured to selectively open and close its respective duct (d1, d2, d3, d4), thus allowing to manage the fluid distribution among the ducts (d1, d2, d3, d4) of the fluidic network.

The first panel (I) illustrates the loading of the sample into the lysing chamber (25), as illustrated by the first arrow (i). A sample container (e.g., a syringe) is connected to the sample inlet port (21), and loads the sample into the lysing chamber (25).

Any extra fluid can be evacuated by the discharge port (24) as illustrated by a second arrow (o), while the solid material remains in the lysing chamber (25), which contains a filter for separating macroscopic materials as food remains, from microscopic materials as the sought micro-organisms. Further details of this filtration can be found in document WO 2023/131714, e.g., pages 8 to 10, or page 15.

During this step of loading, a second valve (27) of the elution duct (d2) and a third valve (28) of the supplementary duct (d3) are closed, as illustrated by the "X" symbol, to prevent the extra fluid to contaminate the sample outlet port (22) and the elution port (23).

The second panel (II) illustrates the rising of the sample, during which the macroscopical material of the sample are discharged from the lysing chamber (25) in order to keep only the sought micro-organisms for the next step of lysing.

During the rinsing step, a rinsing solution is loaded through the elution port (23), as illustrated by a first arrow (i), the solution drags the macroscopic material, then leaves the chip (10) through the discharge port (24), as illustrated by the second arrow (o).

During the rinsing step, a first valve (26) of the loading duct (d1) and the second valve (27) are closed, as illustrated by the "X" symbol, thus guiding the elution solution to the discharge port (24).

The third panel (III) illustrates the lysing step, during which pressure is applied on the lysing chamber (25), as illustrated by a third arrow (p). Given the pressure can lead to a slight oozing from the sample, a valve remains open so that any exuded fluid can go in direction of a duct. Preferably, the third valve (28) remains open so that the exuded fluid goes in direction of the elution duct (d3), while the three other valves (26, 27, 29) are closed, as illustrated by the "X" symbol. Any exuded fluid contained in the elution duct (d3) will be re-collected by the elution solution during the elution step, and will be carried towards the sample outlet port (22). This allows to not lose any sample material.

The pressure applied on the micro-organisms breaks their cells, thus releasing the DNA of the micro-organisms.

The fourth panel (IV) illustrates the elution step, during which the released DNA is brought to the sample outlet port (22), by an elution solution coming from the elution port (23).

During this step, the first valve (26) and the fourth valve (29) are closed, as illustrated by the "X" symbol.

The elution is a critical step of the whole process, as the quantity of elution solution sent into the chip (10) is of a few microliters only, and because the DNA tends to stick to the internal walls of the elution duct (d2).

Even if it not illustrated, the chip (10) can be of the type without valves (26, 27, 28, 29), as disclosed in WO 2023/131714. In this case:
- the sample inlet port (21) is also used as the discharge port (24) during the rinsing step; and as the elution port (23) during the elution step;
- the sample outlet port (22) is also used as the discharge port (24) during the loading step, and as the elution port (23) during the rinsing step.

Because managing pressures, quantities and fluids delivery are very delicate in the field of micro-fluidics, the fluidic network is the shortest possible. This is mostly important for the elution duct (d2), used when the DNA is already released from the micro-organisms and must be conveyed to the elution port (23).

The length of the elution duct (d2) is less than 25mm, preferably less than 20mm. Best results of elution were obtained with elution ducts of length from 10mm to 15mm included.

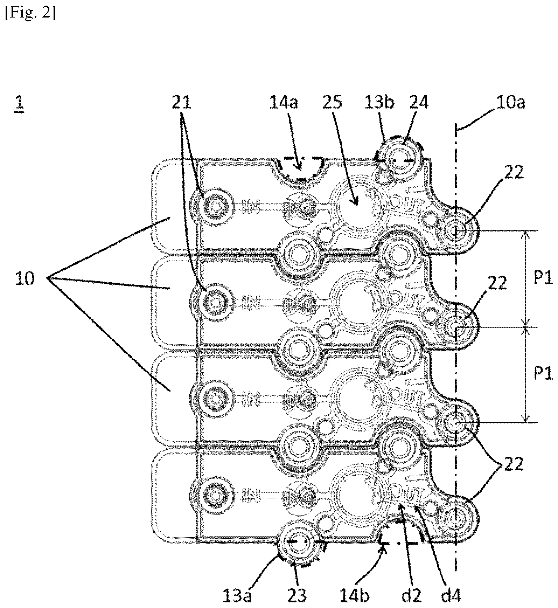

In reference to figure 2, the arrangement of the chips (10) is an array, so that each sample outlet port (22) is disposed on an axis (10a). The sample outlet ports (22) are distant the one from the other by a first pitch (P1) corresponding to the pitch of a standard multi-pipette, e.g., 9mm, the same pitch separating each chip (10). Given the elution ducts (d2) are the same for each chip (10) of the plate (1), the behavior of each chip (10) is equivalent from the one to the other, and each chip (10) provides result of equivalent level. False negative results and erratic behavior, between chips (10) of a same plate (1), are avoided.

To do so, functional parts of a chip (10) overlap the rectangle area corresponding of an adjacent chip (10), in a non-functional area said adjacent chip (10). To that end, the chip (10) comprises, on a first lateral side (10 11), a protrusion (13) resulting from the projection of a functional part, as a port, and a second lateral side (10 12), opposite to the first lateral side (10 11), comprises a concavity (14) matching said protrusion (13).

As a result, two adjacent chips (10) engage together in the manner of a tile pavement, or as puzzle parts. This leads to a decrease of the first pitch (P1), which can be as small as e.g., 14mm: this value corresponds to the widest pitch of common multi-pipettes.

On the illustrated embodiment, the chips (10) comprise four ports (21, 22, 23, 24): given the high number of ports to place on the layout of the chip (10), each chip (10) thus comprises two protrusions (13) and two concavities (14), placed on its lateral sides.

The first lateral protrusion (13a) and the second lateral protrusion (13b) can be on the same lateral side. Preferably, the first lateral protrusion (13a) in on a first lateral side (10 11) and the second lateral protrusion (13b) is on a second lateral side (10 12), opposite the first lateral side (10 11). This allows a better balance of the chip (10) when pipe connectors are plugged into the ports (21, 22, 23, 24) of the chip (10), especially if done by an automatic machine:
the two ports are distributed on each lateral side, so the forces applied by the pipe connectors on said ports tend to compensate themselves relating to a longitudinal direction (L). The chip (10) is better balanced, avoiding its flipping around a transversal direction (T).

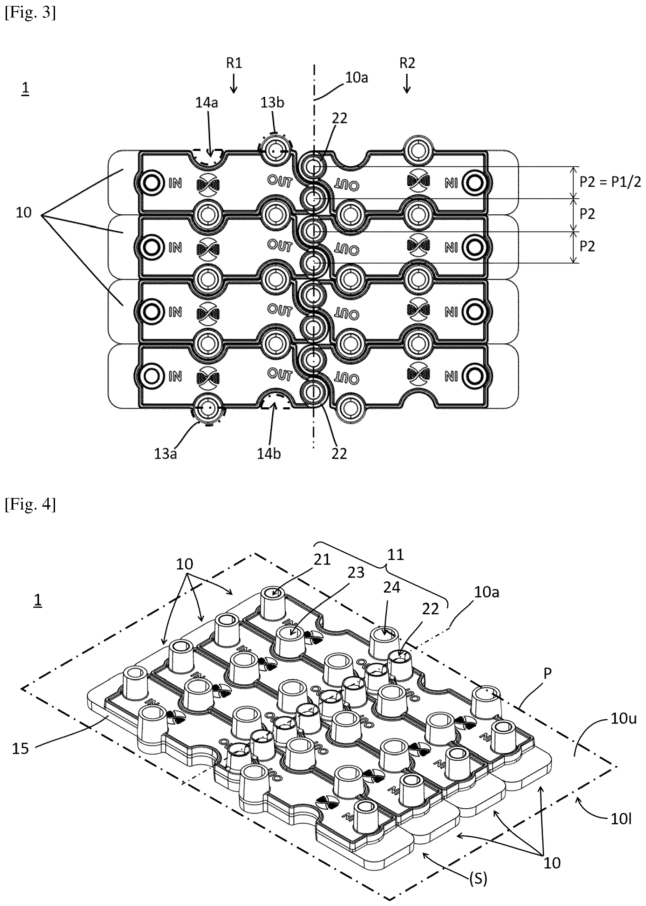

Figure 3 illustrates a preferred embodiment of the invention, wherein the plate (1) comprises two rows of chips (10), each aligned with the axis (10a), but with a first row (R1) disposed on a first side in relation to the axis (10a), and a second row (R2) disposed on a second side in relation to the axis (10a), the chips (10) of the two rows (R1, R2) being disposed head-to-head, in a staggered manner.

The staggered disposition of the chips (10) allows to obtain a plate (1) wherein a second pitch (P2) is further reduced in comparison to the first embodiment: the second pitch (P2) equals the first pitch (P1) divided by two.

The pitch is measured between two consecutives chips (10) in the direction of the axis (10a):
- in the first embodiment, the first pitch (P1) is measured between two adjacent chips (10);
- in the preferred embodiment, the second pitch (P2) is measured between chips (10) placed alternatively on the first row (R1) and on the second row (R2).

In this embodiment, the chips (10) comprise a transversal protrusion (13c), on a transversal side (10 t), and resulting from the projection, in a top view, of the sample outlet port (22). The transversal side (10 t) also comprises a transversal concavity (14c) allowing space for the transversal protrusion (13c) of a facing chip (10).

It must be understood that the engagement of a protrusion (13) and a concavity (14) does not need to be adjusted or to be tight: the main feature is to make the most of the-nonfunctional area of a chip (10). A concavity (14) may leave a gap around its matching protrusion (13).

Preferably, all the chips (10) of the plate (1) are identical. That leads to facing chips (10) being symmetrical, according to a central symmetry around a middle point between their respective sample outlet ports (22).

Figure 4 illustrates the preferred embodiment from an above perspective point of view. This figure shows frangible lines (S) linking the chips (10) of the plate (1). A frangible line (S) allows to separate manually the chips (10) from the plate (1). This may be needed if a test does not require to use all the chips (10) of the plate (1). The laboratory technician can take only the chips (10) he needs from the plate (1), and keep the remaining chips (10) for a later use.

The frangible line (S) can be obtained by any appropriate mean. It can be a score or a notch made for example in a connecting panel (15) of the plate (1). It can be a dotted line obtained by perforation. In the case of a multi-material connecting panel (15), the connecting panel (15) can be mostly made of a first material whereas the frangible line (S) can be made of a second material, weaker than the first material.

When the chips (10) of a plate (1) are all identical and frangible, this allows to use any separated chip (10) indifferently, regardless of the position it had on the plate (1). These interchangeable chips (10) are more convenient to use.

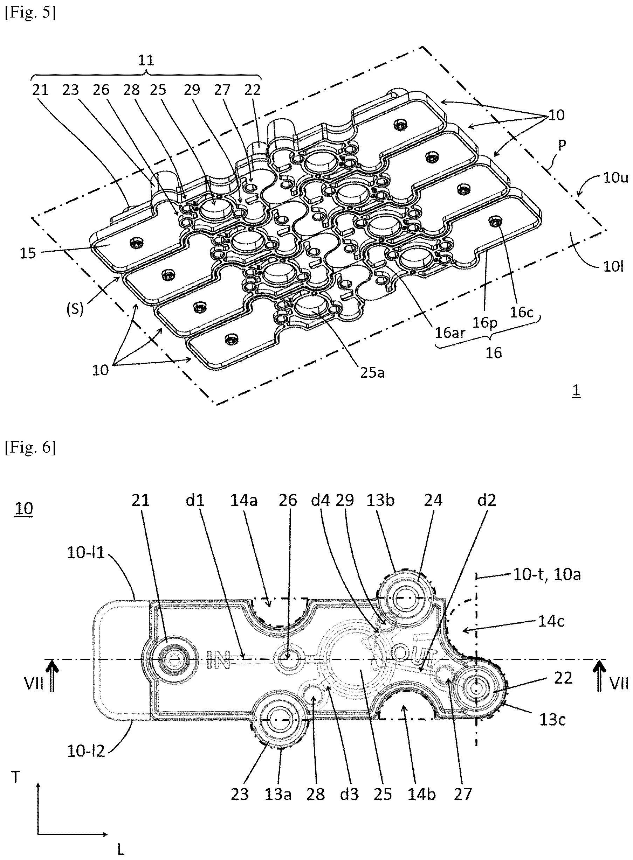

Figure 4 and 5 show that the plate (1) globally extends in a plane (P) which defines an upper face (10u) of the chip (10) and a lower face (101) opposite to the upper face (10u).

Fluidic interfaces (11) of the chips (10) are preferably disposed on each side of the plane (P). This way, fluidic actuators configured to actuate the fluidic interfaces (11) are lesser a constraint when designing the layout of the chips (10): both sides of the chip (10) can be used. The layout of the chip (10), thus the plate (1), can be further reduced.

Preferably, all the ports (21, 22, 23, 24) of the chip (10) are disposed on the upper face (10u), wherein valves access holes (12) are all disposed on the lower face (101). The valves access holes (12) allow an actuator, as a pin, to apply pressure on the valve which is of the type of a deformable membrane.

Having the ports (21, 22, 23, 24) on the upper face (10u) allows:
- a better access to the sample outlet port (22) during pipetting, and avoids spillage;
- to freely place syringes containing the samples on the sample inlet ports (21).

Having all the valve access holes (12) on the lower face (101) of the plate (1) allows to better allocate space for fluidic actuators in an automated machine processing the plate (1), with:
- the pipe connectors on the same side of the plane (P), and
- valve actuators on the same side of the plane (P).

Grouping the fluidic actuators by their category, i.e., pipe connectors and valve actuators, allows to simplify the conception of the automated machine.

Figure 5 also illustrates locating elements (16) allowing to correctly position each chip (10) in a nesting platform of the machine configured for an automated lysing. More precisely, the nesting platform is configured to receive the plate (1), and the nesting platform comprises a bay for each of the chip (10). Each bay comprises locators configured to match with the locating elements (16) of the chip (10), and correctly position the chip (10) for the automated process.

This way:
- a complete plate (1) is correctly positioned in the nesting platform; and
- each chip (10) which has been separated from the plate (1), thanks to the frangible line (S), is also correctly positioned in the nesting platform;
provided that each chip (10) of the plate (1) is held by the locators of each bay, through the locating elements (16).

In order to ease the positioning of the chips (10) or the plate (1) when laying them on the nesting platform, the locating elements (16) are disposed on the lower face (101) of the chip (10) plane (P). Gravity maintains the chips (10) or the plate (1) in a correct positioning.

In the preferred illustrated embodiment, the locating elements (16) comprise a centering pin (16c), a planar support (16p) and an anti-rotation device (16ar). This combination provides an isostatic positioning, which avoids a deformation of the chip (10) or of the plate (1).

Other types of locating elements (16) can be used. An isostatic positioning is preferred.

In particular, a ridge defines both a structural reinforcement of the chip (10) and the planar support (16p). In this way, the design of the chip (10) is simpler, and the manufacturing of the chip (10) is cheaper. The ridge may be peripheral.

Preferably, the locating elements (16) are disposed with a dissymmetric pattern, in order to prevent the mounting of a chip (10) in an inverted position. This provides "mistake-proofing" locating elements (16), because a chip (10) cannot be mounted in the nesting platform in a different way than the one expected.

In the illustrated example:
- the centering pin (16c) is offset in a longitudinal direction (L) of the chip (10),
- the anti-rotation device (16ar) is offset both in the longitudinal direction and a transversal direction (T) of the chip (10).

Figure 6 illustrates in details the components of the preferred chip (10) from the plate (1), and summarizes the afore-mentioned features.

In order to avoid cross contamination during the lysing of the sample, the chip (10) comprises the following separate ports: a sample inlet port (21), an elution port (23), a rinsing port, and sample outlet port (22).

The fluidic network comprises the following ducts:
- a loading duct (d1), linking the sample inlet port (21) to the lysing chamber (25);
- a elution duct (d2), linking the lysing chamber (25) to the sample outlet port (22);
- a supplementary duct (d3), linking the elution port (23) to the lysing chamber (25);
- a discharge duct (d4), linking the lysing chamber (25) to the rinsing port.

In addition to the ports, the fluidic interfaces (11) comprise the following valves:
- a first valve (26), on the loading duct (d1);
- a second valve (27), on the elution duct (d2);
- a third valve (28), on the supplementary duct (d3);
- a fourth valve (29), on the discharge duct (d4),
each valve (26, 27, 28, 29) being configured to be actuated between an open state allowing the flow of fluid through its duct (d1, d2, d3, d4), and a closed state preventing any flowing through its duct (d1, d2, d3, d4).

According to the different phases of the mechanical lysing, and the configuration to adopt for the fluidic network, each valve (26, 27, 28, 29) can be actuated through the valve access hole (12).

On the top view of figure 6, the shapes of the protrusions (13) and the concavities (14) are clearly visible.

The elution port (23) provides the first lateral protrusion (13a), on the first lateral side (1011) of the chip (10). The first lateral concavity (14a), antagonist of the first lateral protrusion (13a), is disposed on the second lateral side (10 12) of the chip (10). The first lateral concavity (14a) is configured to receive the first lateral protrusion (13a) of an adjacent chip (10), placed alongside with the illustrated chip (10), on the second lateral side (10 12).

The discharge port (24) provides the second lateral protrusion (13b), on the second lateral side (1012) of the chip (10). The second lateral concavity (14b), antagonist of the second lateral protrusion (13b), is disposed on the first lateral side (10 11) of the chip (10). The second lateral concavity (14b) is configured to receive the second lateral protrusion (13b) of another adjacent chip (10), placed alongside with the illustrated chip (10), on the first lateral side (10 11).

The sample outlet port (22) provides the transversal protrusion (13c), on a transversal side (10 t) of the chip (10). The transversal concavity (14c), antagonist of the transversal protrusion (13c), is disposed on the same transversal side (10 t). The transversal concavity (14c) is conformed to receive the transversal protrusion (13c) of a facing chip (10), placed in a staggered manner with the illustrated chip (10), on the other side of the axis (10a) which is aligned with the transversal side (10 t).

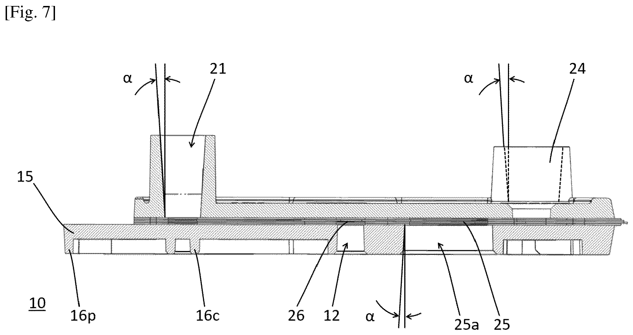

Figure 7 illustrates a cross section view of the chip (10), illustrating a conicity (α) of fluidic interfaces (11). The conicity (α) allows a better self-positioning of a fluidic actuator actuating the chips (10) of the plate (1), notably during the automated process of the plate (1). The conicity (α) helps guiding the actuator.

On figure 7, not all conicities (α) are illustrated in order to prevent overfilling figure 7.

Preferably:
- the conicity (α) of a pestle access hole (25a) for a pestle eases the introduction of said pestle for the mechanical lysing of the sample;
- the conicity (α) of the valve access hole (12) eases the introduction of actuators, as pins, for actuating its valve (26, 27, 28, 29).

As for the ports (21, 22, 23, 24), the conicity (α) provides two advantageous effects: it eases the introduction of the pipe connector, or syringe, intended to plugged on its port, but it also ensures the sealing of this connection through a radial tightness obtained through the diameter increase of the conicity (α).

The illustrated ports (21, 22, 23, 24) present an internal conicity (α), because their interfaces are configured to plug into said ports (21, 22, 23, 24). The conicity (α) can also be external, for interfaces capping said ports (21, 22, 23, 24), instead of plugging into said ports (21, 22, 23, 24).

Furthermore, the chips (10), and the plate (1) comprising the chips (10), can be shaped differently from the examples given without departing from the scope of the invention, which is defined by the claims.

In addition, the technical features of the various embodiments and variants mentioned above can be combined with one another, either in whole or in part. In this way, the chips (10), and the plate (1) comprising the chips (10), can be adapted in terms of cost, functionality and performance.

## Claims

1. Plate (1) comprising at least two chips (10) designed for receiving a sample to be lysed, each chip (10) featuring a fluidic network comprising fluidic interfaces (11) which includes:
- ports, including:
- an elution port (23) linked to a lysing chamber (25),
- a sample outlet port (22) configured for collecting a lysed sample,
- at least one duct, including an elution duct between the lysing chamber (25) and the sample outlet port (22), for transferring a lysed sample from the lysing chamber (25); the chips (10) being disposed so that:
- the sample outlet port (22) of each chip (10) coincides with an axis (10a), and
- the sample outlet ports (22) of two adjacent chips (10) are spaced apart with a pitch, ***characterised in that*** the elution duct is identical for each chip (10) of the plate (1).

2. The plate (1) of claim 1, wherein the supplementary duct is identical for each chip (10) of the plate (1), and preferably the fluidic networks of each chip (10) are identical.

3. The plate (1) of any preceding claim, wherein the chips (10) are aligned with the axis (10a) and regularly spaced apart, and a top-view projection of the chips (10) features:
- protrusions (13) resulting from ports (22, 23), and
- concavities (14) configured to match the protrusions of the top-view projections of an adjacent chip (10), in the manner of a tiling.

4. The plate (1) of claim 3, wherein a first lateral side of the chip (10) comprises a first lateral protrusion (13a) resulting from the elution port (23), and a second lateral side (10 12), opposite to the first side, comprises a first lateral concavity (14a) configured to match the first lateral protrusion (13a).

5. The plate (1) of claim 4, wherein the first lateral side comprises a second lateral protrusion (13b) resulting from a discharge port (24), and the second lateral side (10 12) comprises a second lateral concavity (14b) configured to match the second lateral protrusion (13b).

6. The plate (1) of claim 3 to 5, wherein the chips (10) are arranged with a staggered layout on each side of the axis (10a).

7. The plate (1) of claim 6, wherein a transversal side of the chip (10) comprises a transversal protrusion (13c) resulting from the sample outlet port (22), and a transversal concavity (14c) configured to match the transversal protrusion (13c).

8. The plate (1) of any preceding claim, wherein two adjacent chips (10) are linked together by a frangible line, allowing to manually separate the chips (10) from the plate (1).

9. The plate (1) of any preceding claim, wherein the chip (10) features locating elements (16), configured to match a nesting platform (30) of a machine (2) during an automated lysing.

10. The plate (1) of claim 6, wherein the locating elements (16) are located on a lower face (101) of the chips (10).

11. The plate (1) of any claim from 6 to 8 wherein the locating elements (16) are disposed on the chip (10) with a dissymmetry pattern.

12. The plate (1) of any preceding claim, wherein the fluidic interfaces (11) are dispatched on both sides of the chip (10) which is globally planar.

13. The plate (1) of claim 12, wherein the fluidic interfaces (11) comprise:
- a valve (26) configured to selectively open and close said at least one duct of the fluidic network, and
- a valve access hole (12) allowing an actuation of the valve (26),
and the ports (22,23) are located on an upper face (10u) of the plate (1), whereas the valve access hole (12) is located opposite to the upper face (10u).

14. The plate (1) of any preceding claim, wherein a fluidic interface (11) features a conical shape protruding from a chip plane (P) defined by the chip (10), presenting a distal section, away from the plane (P), which is inferior to a proximal section, close to the plane (P), and the conical shape is configured to guide a fluidic actuator actuating the fluidic interface (11).

15. The plate (1) of claim 14, wherein the fluidic actuator is a pipe connector designed to be plugged on one of the ports of the fluidic network, and the conical shape is configured to seal the connection between said port and the connector.
